# EUROPEAN PATENT APPLICATION

(11) **EP 4 782 049 A1**
(43) Date of publication of application: **29.07.2026**
(21) Application number: 26153146.1
(22) Date of filing: 21.01.2026
(51) Int. Cl.: A61M 60/117, A61M 60/253, A61M 60/258, A61M 60/268, A61M 60/274, A61M 60/829, A61M 60/835, A61M 60/857, A61M 60/896, A61M 1/36

(54) **BLOOD PUMP**

(30) Priority: 22.01.2025 US 202563748246 P
(71) Applicant: Abiomed, Inc., Danvers, MA 01923 (US)
(72) Inventor: KIRKPATRICK, Robert, Danvers, MA 01923 (US)
(74) Representative: Wittmer, Maximilian

(57) **Abstract**

A device, system, and method of use are provided. The device includes a cannula having a lumen extending therethrough, an inflow cage coupled to one end of the cannula and an outflow cage coupled to the other end of the cage. The inflow cage includes an inflow check valve that is configured to allow blood to flow into the lumen only when a pressure inside the lumen is less than a pressure of blood external to the inflow check valve. The outflow cage includes an outflow check valve that is configured to allow blood to flow out of the lumen through at least one outlet only when a pressure in the lumen is greater than a pressure of blood external to the outflow check valve. A catheter is operably coupled the outflow cage. A pump capable of generating positive and negative pressures is operably coupled to the lumen.

## Description

### CROSS-REFERENCE TO RELATED APPLICATIONS

The present application claims priority to U.S. Provisional Patent Application No. 63/748,246, filed January 22, 2025, the contents of which are incorporated herein in its entirety.

### TECHNICAL FIELD

The present application is drawn to blood pumps, and specifically, to blood pumps configured to be inserted into or through blood vessels in a body.

### BACKGROUND

Blood pump assemblies, such as intracardiac or intravascular blood pumps may be introduced in the heart to deliver blood from the heart into an artery. Such mechanical circulatory support devices are often introduced to support the function of the heart after a patient suffers a cardiac episode. One such class of devices is the set of devices known as the "Impella" heart pump. Some blood pump assemblies may be introduced percutaneously through the vascular system during a cardiac procedure. Specifically, blood pump assemblies can be inserted via a catheterization procedure through the femoral artery or the axillary/subclavian artery, into the ascending aorta, across the valve and into the left ventricle. The inserted blood pump assembly may be configured to pull blood from the left ventricle of the heart through a cannula and expels the blood into the aorta. A blood pump assembly may also be configured to pull blood from the inferior vena cava and to expel blood into the pulmonary artery. Some mechanical circulatory support devices are powered by an on-board motor, while others are powered by an external motor and a drive cable.

### BRIEF SUMMARY

In various aspects, a blood pump may be provided. The blood pump may include a cannula having a lumen therethrough. The blood pump may include an inflow cage coupled to a distal end of the cannula. The inflow cage may include an inflow check valve. The inflow check valve may be configured to allow blood to flow into the lumen only when a pressure inside the lumen is less than a pressure of blood external to the inflow check valve. The blood pump may include an outflow cage coupled to a proximal end of the cannula. The outflow cage may include an outflow check valve. The outflow check valve may be configured to allow blood to flow out of the lumen through at least one outlet only when a pressure in the lumen is greater than a pressure of blood external to the outflow check valve. The blood pump may include a catheter operably coupled to a proximal end of the outflow cage. The blood pump may include a pump capable of generating positive and negative pressures coupled to the lumen.

The pump may be coupled to a proximal end of the catheter. The pump may be configured to be positioned external to a patient during operation. The pump may be a positive displacement pump. The pump may be a piston pump. The piston pump may include a piston and a cylinder. The piston pump may be configured such that the upstroke draws at least some blood passing through the cannula into the cylinder.

The blood pump may include a purge pump configured to provide a purge fluid (such as a saline purge fluid) to the pump. The blood pump may include a distal extension assembly operably connected to a distal end of the inflow cage. The distal extension assembly may include a check valve configured to allow blood to flow through a distal end of the inflow cage.

The inflow check valve may include a membrane operatively coupled to an internal surface of the inflow cage. The membrane may be integrally formed with the inflow check valve. The inflow check valve may include an elastomer reinforced with nitinol.

The outflow check valve may include a membrane coupled to an external surface of the outflow cage. The membrane may be integrally formed with the outflow check valve. The membrane may extend over the at least one outlet and at least a portion of the catheter. The outflow check valve may include an elastomer reinforced with nitinol.

The blood pump may include a sleeve coupled to an external surface of the blood pump. The sleeve may be configured to extend over the outflow check valve and at least a portion of the catheter. The sleeve may have a proximal outlet configured to allow blood to exit the sleeve.

The catheter may include an inner surface having a low-friction coating.

The blood pump may be configured to transfer blood through the cannula at a maximum rate of 1-5 L/min through the cannula.

The blood pump may include baffles proximal to the outlet. The baffles may be configured to dampen and smooth a flow of blood from the at least one outlet.

In various aspects a system may be provided. The system may include a blood pump as disclosed herein. The system may include a controller operably connected to the blood pump.

In various aspects, a method for using a blood pump may be provided. The method may include providing a blood pump as disclosed herein. The method may include priming the blood pump. The method may include inserting the blood pump into a patient through at least one blood vessel.

The blood pump may include a piston pump, with a piston and a cylinder. The method may include repeatedly performing at least two steps. The two steps may include moving the piston so as to generate a negative pressure, closing the outlet check valve and opening the inlet check valve, drawing blood through the inlet check valve and into the cylinder. The two steps may include moving the piston so as to generate a positive pressure, closing the inlet check valve and opening the outlet check valve, forcing blood out of the cylinder and through the outlet check valve.

The method may include causing a purge fluid to flow through the pump. A flow rate of the purge fluid may be less than a flow rate of blood flowing through the blood pump. The method may include allowing or causing a check valve in a distal extension assembly coupled to a distal end of the inflow cage to open, allowing blood to enter the inflow cage and move any blood at the distal end of the inflow cage towards the cannula.

### BRIEF DESCRIPTION OF FIGURES

The accompanying drawings, which are incorporated in and constitute a part of this specification, illustrate embodiments of the present invention and, together with a general description of the invention given above, and the detailed description of the embodiments given below, serve to explain the principles of the present invention.
Figure 1 is an illustration of an embodiment of a system.
Figure 2 is an illustration of a cross-sectional view of an embodiment of an inflow cage with an inflow check valve.
Figure 3 is an illustration of a cross-sectional view of an embodiment of an outflow cage with an outflow check valve.
Figure 4 is an illustration of an embodiment of a device with a sleeve.
Figure 5 is an illustration of a cross-sectional view of an embodiment of a sleeve surrounding an outflow cage.
Figure 6 is an illustration of an embodiment of a device positioned partially within a heart.
Figure 7 is a flowchart of a method.

It should be understood that the appended drawings are not necessarily to scale, presenting a somewhat simplified representation of various features illustrative of the basic principles of the invention. The specific design features of the sequence of operations as disclosed herein, including, for example, specific dimensions, orientations, locations, and shapes of various illustrated components, will be determined in part by the particular intended application and use environment. Certain features of the illustrated embodiments have been enlarged or distorted relative to others to facilitate visualization and clear understanding. In particular, thin features may be thickened, for example, for clarity or illustration.

### DETAILED DESCRIPTION

The following description and drawings merely illustrate the principles of the invention. It will thus be appreciated that those skilled in the art will be able to devise various arrangements that, although not explicitly described or shown herein, embody the principles of the invention and are included within its scope. Furthermore, all examples recited herein are principally intended expressly to be only for illustrative purposes to aid the reader in understanding the principles of the invention and the concepts contributed by the inventor(s) to furthering the art and are to be construed as being without limitation to such specifically recited examples and conditions. Additionally, the term, "or," as used herein, refers to a non-exclusive or, unless otherwise indicated (e.g., "or else" or "or in the alternative"). Also, the various embodiments described herein are not necessarily mutually exclusive, as some embodiments can be combined with one or more other embodiments to form new embodiments.

The numerous innovative teachings of the present application will be described with particular reference to the presently preferred exemplary embodiments. However, it should be understood that this class of embodiments provides only a few examples of the many advantageous uses of the innovative teachings herein. In general, statements made in the specification of the present application do not necessarily limit any of the various claimed inventions. Moreover, some statements may apply to some inventive features but not to others. Those skilled in the art and informed by the teachings herein will realize that the invention is also applicable to various other technical areas or embodiments.

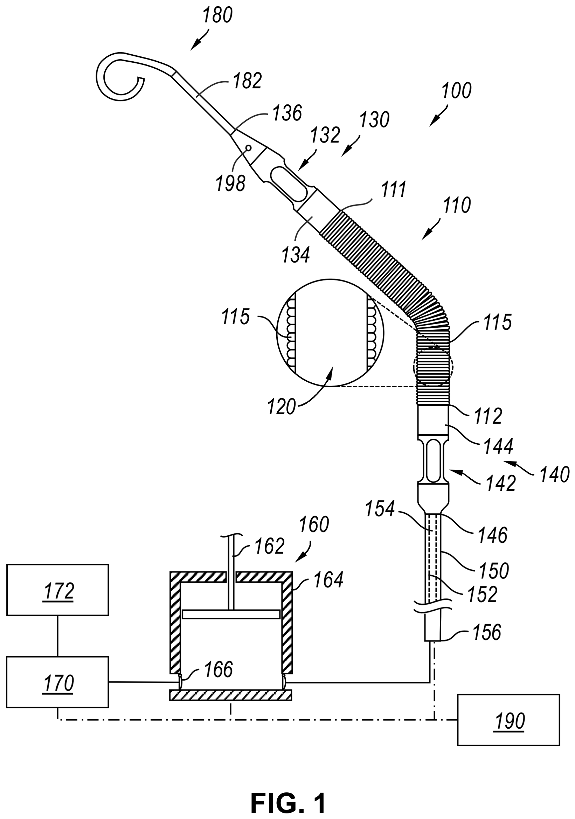

Referring to FIG. 1, in various aspects, a blood pump **100** may be provided. The blood pump may include a cannula 110. The cannula may have sidewalls **115** defining a lumen **120** extending therethrough, from a distal end **111** to a proximal end 112. As will be appreciated, blood may be transferred through cannula, from an inflow to an outflow connected thereto.

In some embodiments, the blood pump may include an inflow cage **130** coupled to a distal end of the cannula. The inflow cage may include one or more inlets 132. The inflow cage may include an inflow check valve 134. The inflow check valve may be configured to allow blood to flow into the lumen only when a pressure inside the lumen is less than a pressure of blood external to the inflow check valve. The inflow check valve may be disposed proximal to the one or more inlets.

The blood pump may include an outflow cage **140** coupled to a proximal end of the cannula. The outflow cage may include one or more outlets 142. The outflow cage may include an outflow check valve 144. The outflow check valve may be configured to allow blood to flow out of the lumen through at least one outlet only when a pressure in the lumen is greater than a pressure of blood external to the outflow check valve.

The blood pump may include a catheter **150** operably coupled to a proximal end **146** of the outflow cage. The catheter may include an inner surface **152** (e.g., of a lumen **154** extending at least partially through the catheter). The inner surface may have a low-friction coating.

The blood pump may include a pump **160** coupled to the lumen. The pump may be capable of / configured to generate positive and negative pressures. The pump may be disposed proximal to the catheter. The pump may be coupled to a proximal end **156** of the catheter. The pump may be configured to be positioned external to a patient during operation.

The pump may be a positive displacement pump.

The positive displacement pump may be a gear pump, piston pump, or any of the other well-known types of positive displacement pumps. As used herein, the term "positive displacement pump" is intended to refer to any of a variety of pumps capable of developing high discharge pressure even at low speeds, approaching zero speed.

The pump may be a piston pump. The piston pump may include a piston 162 and a cylinder 164. The pump may include one or more valves 166 configured to control the flow of fluid through the pump. As is known in the art, piston pumps have an upstroke (upstroke draw, creating negative pressure and drawing fluid into the cylinder) and a downstroke (creating positive pressure and forcing fluid out of the cylinder). The piston pump may be configured such that the upstroke draws at least some blood passing through the cannula into the cylinder.

The blood pump may include a purge pump 170 configured to provide a purge fluid (e.g., from a purge fluid source 172) to the pump. The purge fluid may be any appropriate purge fluid, such as a saline (or saline-based) purge fluid. The saline purge fluid may be provided at a constant flow rate. The saline purge fluid may be provided intermittently.

The blood pump may include a distal extension assembly 180 operably connected to a distal end 136 of the inflow cage. The distal extension assembly may include a check valve 182 configured to allow blood to flow through the distal extension assembly and into the distal end of the inflow cage.

A controller 190 may be operably coupled to the blood pump 100. The controller may be operably coupled to the pump 160 and the purge fluid pump 170. The controller may be configured to operate the blood pump so as to create a pulsatile flow through the one or more outlets 142 of the outflow cage 140.

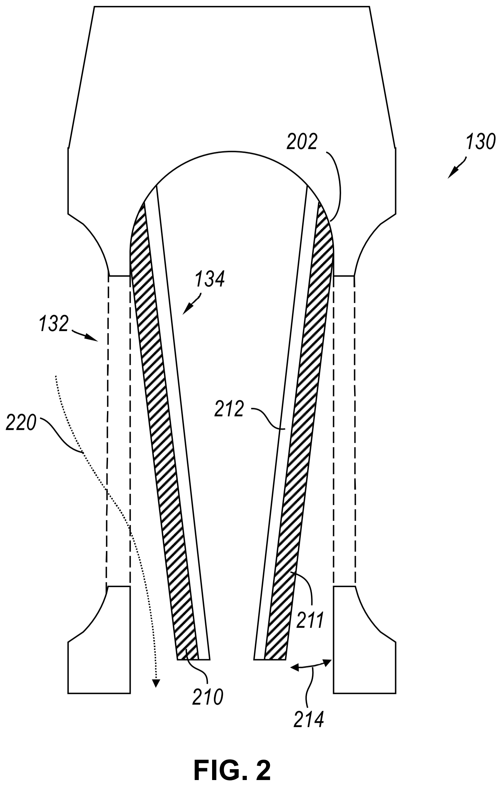

Referring to FIG. 2, in some embodiments, the inflow check valve 134 may include a membrane 210 operatively coupled to an internal surface 202 of the inflow cage 130. The membrane may be integrally formed with the inflow check valve. The membrane may be an elastomer. In some embodiments the inflow check valve may include an elastomer 211 reinforced with one or more reinforcing layers 212. The elastomer may be a biocompatible elastomer. The elastomer may be, e.g., a polyurethane. The reinforcing layers may include a metal or metal alloy, such as nitinol. The inflow check valve may be disposed radially inward from a blood flow inlet 132, such that when the pressure external to the inflow check valve is greater than the internal pressure, blood may form a pathway 220 from external to the check valve, through the inlet, through a gap 214 formed between the membrane / elastomer and the inner surface of the inflow cage, and then into the cannula (not shown).

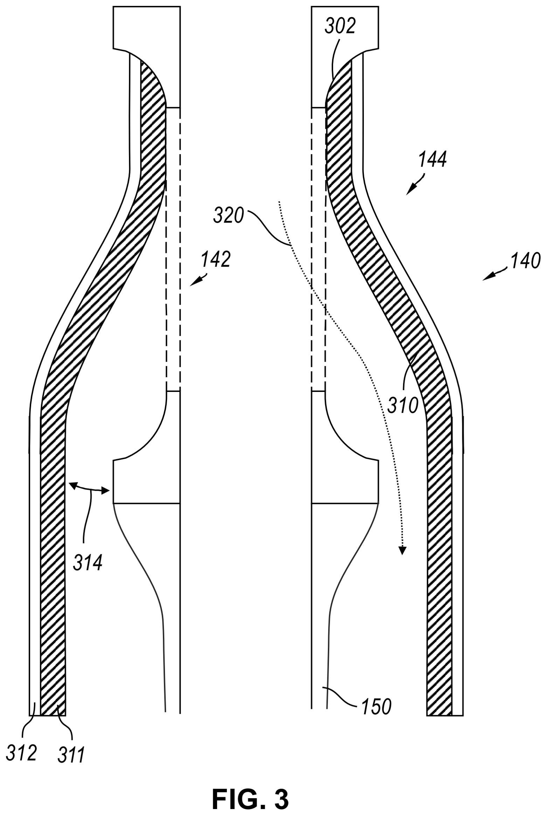

Referring to FIG. 3, in some embodiments, the outflow check valve 144 may include a membrane 310 coupled to an external surface 302 of the outflow cage 140. The membrane may be integrally formed with the outflow check valve. The membrane may extend over the at least one outlet 342. The membrane may extend over the at least one outlet and at least a portion of the catheter 150. The outflow check valve may include an elastomer **311** reinforced with a reinforcing layer 312. The elastomer may be, e.g., a polyurethane. The reinforcing layers may include a metal or metal alloy, such as nitinol. The outflow check valve may be disposed radially outward from a blood flow outlet **142,** such that when the pressure internal to the outflow check valve is greater than the external pressure, blood may form a pathway **320** from internal to the check valve, through the outlet, and through a gap **314** formed between the membrane / elastomer and the outer surface of the outflow cage.

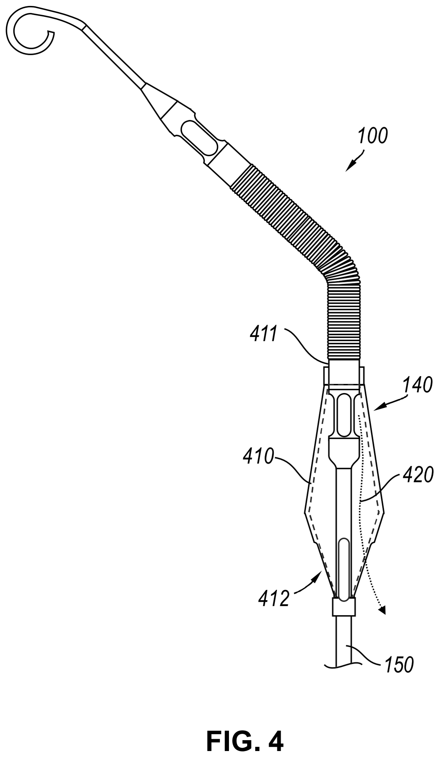

Referring to FIG. 4, the blood pump may include a sleeve **410** coupled to an external surface **411** of the blood pump. The sleeve may be a polymer sleeve. The sleeve may be, e.g., a polyurethane. The sleeve may be configured to extend over the outflow check valve **140** and at least a portion of the catheter 150. The sleeve may have a proximal outlet **412** configured to allow blood to exit the sleeve. That is, the sleeve may be disposed radially outward from the outflow check valve, such that blood flowing out of the blood flow outlet **142** will pass through the sleeve before leaving the sleeve at the proximal outlet 412.

The blood pump may be configured to transfer blood through the cannula at a maximum rate of 1-7 L/min through the cannula. The blood pump may be configured to transfer blood through the cannula at a maximum rate of 1-5 L/min through the cannula. In some embodiments, the blood pump may be configured to transfer blood through the cannula at a minimum rate of 0.5-2.5 L/min through the cannula.

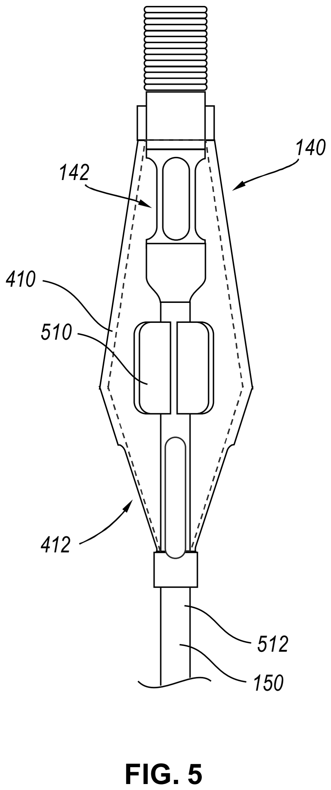

Referring to FIG. 5, the blood pump may include baffles **510** proximal to the outlet . The baffles may be configured to dampen and smooth a flow of blood from the at least one outlet **142** of the outflow check valve 140. In some embodiments, each baffle may be coupled to an external surface **512** of the catheter. In some embodiments, each baffle may be disposed between the at least one outlet **142** and the proximal outlet(s) 412. In some embodiments, each baffle may be expandable (e.g., having a compressed configuration and an expanded configuration. Such techniques are known in the art; for example, an outer sleeve could keep the baffles in a compressed form while the device is being inserted into a patient, and the outer sleeve could be retracted to allow the baffles to expand.

In various aspects a system may be provided. The system may include an embodiment of a blood pump as disclosed herein. The system may include a controller operably connected to the blood pump. The controller may be configured to control the pump 160. The controller may be configured to receive information from one or more sensors **198,** which may be disposed in or on the blood pump, such as in or on the cannula, the outflow cage, and/or the inflow cage. The controller may be configured to control the purge fluid pump 170.

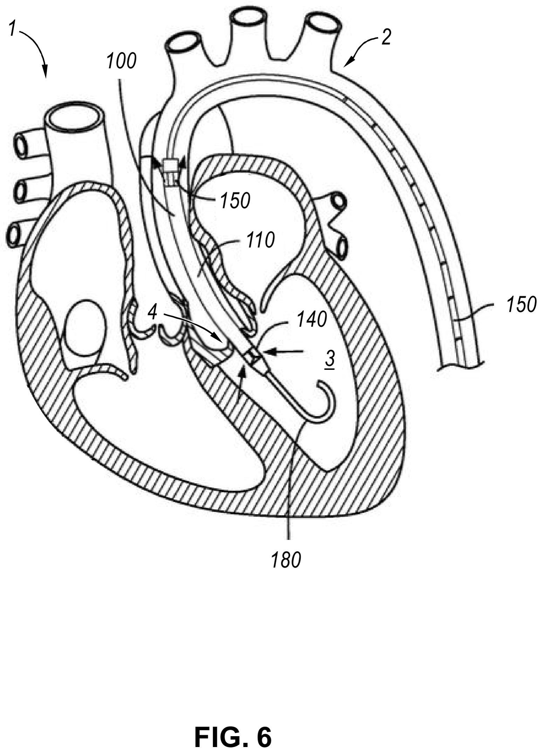

Referring to FIG. 6, the disclosed blood pumps and systems may be introduced into a patient. For example, a blood pump **100** may be introduced to a blood vessel, such as aorta 2. The blood pump may be advanced through the blood vessel, with or without use of a guidewire, and allowing at least a part of the blood pump to pass through, *e.g,,* at least one valve **4,** until the blood pump **100** is disposed in a desired location (such as a left ventricle 3 of a heart 1). In some embodiments, the inlet(s) may be in a first portion of the patient (e.g., left ventricle 3) while the outlets are in a section portion of the patient (e.g., aorta 2), with a valve between inlet and outlet.

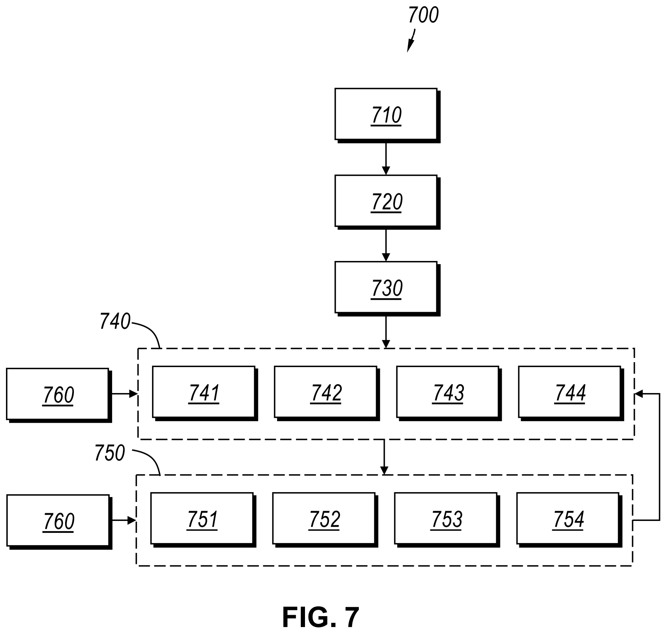

Referring to FIG. 7, In various aspects, a method for using a blood pump may be provided. The method **700** may include providing **710** an embodiment of a blood pump **100** as disclosed herein.

The method may include priming **720** the blood pump. This may include priming pump **160.** Any appropriate technique can be utilizing for priming the pump may be utilized. Priming may include providing a fluid the patient can accept (which may be the patient's own blood or replacement fluids) into the blood pump such that substantially no air remains in lumens the patient's blood vessels will be exposed to.

The method may include inserting **730** the blood pump into a patient through at least one blood vessel (see, e.g., FIG. 6).

As disclosed herein, the blood pump may include a piston pump, with a piston and a cylinder. The method may include repeatedly performing at least two broad steps, and blood drawing step **740** and a blood forcing step **750.** The blood drawing step may include moving **741** the piston **162** so as to generate a negative pressure, closing **742** the outlet check valve **150** and opening **743** the inlet check valve **140,** drawing **744** blood through the inlet check valve **140** and into the cylinder **164.** The blood drawing step may include moving **751** the piston **162** so as to generate a positive pressure, closing **752** the inlet check valve **140** and opening **753** the outlet check valve **150,** forcing **754** blood out of the cylinder **164** and through the outlet check valve **150.** In some embodiments, there may be no delay between the blood drawing and blood forcing steps. In some embodiments, there may be a small delay (e.g., 0 seconds < t ≤ 0.5 second). In some embodiments, there may be no delay between one cycle and a second cycle. In some embodiments, there may be a small delay (e.g., 0 seconds < t ≤ 0.5 second).

The method may include causing **760** a purge fluid to flow through the pump **160.** This may include causing at least some purge fluid to flow through the blood pump (and out through the one or more outlet(s) **142.** A flow rate of the purge fluid may be less than a flow rate of blood flowing through the blood pump. In some embodiments, a flow rate of the purge fluid may be no more than 5% of a flow rate of blood flowing through the blood pump. In some embodiments, a flow rate of the purge fluid may be no more than 10% of a flow rate of blood flowing through the blood pump. In some embodiments, a flow rate of the purge fluid may be no more than 20% of a flow rate of blood flowing through the blood pump.

The method may include allowing or causing **770** a check valve **182** in a distal extension assembly **180** coupled to a distal end of the inflow cage to open, allowing blood to enter the inflow cage and move any blood at the distal end of the inflow cage towards the cannula.

Various modifications may be made to the systems, methods, apparatus, mechanisms, techniques, and portions thereof described herein with respect to the various figures, such modifications being contemplated as being within the scope of the invention. For example, while a specific order of steps or arrangement of functional elements is presented in the various embodiments described herein, various other orders/arrangements of steps or functional elements may be utilized within the context of the various embodiments. Further, while modifications to embodiments may be discussed individually, various embodiments may use multiple modifications contemporaneously or in sequence, compound modifications and the like.

Specific embodiments can be understood as described below.

In a first embodiment, a blood pump may be provided. The blood pump may include: (i) a cannula having a lumen therethrough; (ii) an inflow cage coupled to a distal end of the cannula, the inflow cage comprising an inflow check valve that is configured to allow blood to flow into the lumen only when a pressure inside the lumen is less than a pressure of blood external to the inflow check valve; (iii) an outflow cage coupled to a proximal end of the cannula, the outflow cage comprising an outflow check valve that is configured to allow blood to flow out of the lumen through at least one outlet only when a pressure in the lumen is greater than a pressure of blood external to the outflow check valve; (iv) a catheter operably coupled to a proximal end of the outflow cage; and (v) a pump capable of generating positive and negative pressures operably coupled to the lumen.

In a second embodiment, based on the first embodiment, the pump is coupled to a proximal end of the catheter.

In a third embodiment, based on the second embodiment, the pump is configured to be positioned external to a patient during operation.

In a fourth embodiment, based on the third embodiment, the pump is a piston pump.

In a fifth embodiment, based on the fourth embodiment, the piston pump comprises a piston and a cylinder, and the piston pump is configured such that an upstroke draws at least some blood passing through the cannula into the cylinder.

In a sixth embodiment, based on any one of the first through fifth embodiments, the blood pump further includes a purge pump configured to provide a purge fluid to the pump.

In a seventh embodiment, based on the sixth embodiment, the pump is a positive displacement pump.

In an eighth embodiment, based on any one of the first through seventh embodiments, the blood pump further includes a distal extension assembly operably connected to a distal end of the inflow cage.

In a ninth embodiment, based on the eighth embodiment, the distal extension assembly comprises a check valve configure to allow blood to flow through a distal end of the inflow cage.

In a tenth embodiment, based on any one of the first through ninth embodiments, the inflow check valve comprises a membrane operatively coupled to an internal surface of the inflow cage.

In an eleventh embodiment, based on the tenth embodiment, the membrane is integrally formed with the inflow check valve.

In a twelfth embodiment, based on any one of the first through the eleventh embodiments, the inflow check valve comprises an elastomer reinforced with nitinol.

In a thirteenth embodiment, based on any one of the first through the twelfth embodiments, the outflow check valve comprises a membrane coupled to an external surface of the outflow cage.

In a fourteenth embodiment, based on the thirteenth embodiment, the membrane is integrally formed with the outflow check valve.

In a fifteenth embodiment, based on the thirteenth or fourteenth embodiment, the membrane extends over the at least one outlet and at least a portion of the catheter.

In a sixteenth embodiment, based on any one of the first through fifteenth embodiments, the outflow check valve comprises an elastomer reinforced with nitinol.

In a seventeenth embodiment, based on any one of the first through sixteenth embodiments, the blood pump further includes a sleeve coupled to an external surface of the blood pump, the sleeve being configured to extend over the outflow check valve and at least a portion of the catheter, the sleeve having a proximal outlet configured to allow blood to exit the sleeve.

In an eighteenth embodiment, based on any one of the first through seventeenth embodiments, the catheter comprises an inner surface having a low-friction coating.

In a nineteenth embodiment, based on any one of the first through eighteenth embodiments, the blood pump is configured to transfer blood through the cannula at a maximum rate of 1-5 L/min through the cannula.

In a twentieth embodiment, based on any one of the first through nineteenth embodiments, the blood pump further includes baffles proximal to the at least one outlet, the baffles configured to dampen and smooth a flow of blood from the at least one outlet.

In a twenty-first embodiment, a system may be provided, where the system includes: (i) a blood pump of any one of the first through twentieth embodiments; and (ii) a controller operably connected to the blood pump.

In a twenty-second embodiment, a method for using a blood pump may be provided, the method including: (i) providing a blood pump of any one of the first through twentieth embodiments; (ii) priming the blood pump; and (iii) inserting the blood pump into a patient through at least one blood vessel.

In a twenty-third embodiment, based on the twenty-second embodiment, the blood pump comprises a piston pump including a piston and a cylinder, and where the method further includes repeatedly: (i) moving the piston so as to generate a negative pressure, closing the outflow check valve and opening the inflow check valve, drawing blood through the inflow check valve and into the cylinder; and (ii) moving the piston so as to generate a positive pressure, closing the inflow check valve and opening the outflow check valve, forcing blood out of the cylinder and through the outflow check valve.

In a twenty-fourth embodiment, based on the twenty-second or twenty-third embodiment, the method may further include causing a purge fluid to flow through the pump, a flow rate of the purge fluid being less than a flow rate of blood flowing through the blood pump.

In a twenty-fifth embodiment, based on any one of the twenty-second through the twenty-fourth embodiments, the method may further include causing a check valve in a distal extension assembly coupled to a distal end of the inflow cage to open, allowing blood to enter the inflow cage and move any blood at the distal end of the inflow cage towards the cannula.

Although various embodiments which incorporate the teachings of the present invention have been shown and described in detail herein, those skilled in the art can readily devise many other varied embodiments that still incorporate these teachings. Thus, while the foregoing is directed to various embodiments of the present invention, other and further embodiments of the invention may be devised without departing from the basic scope thereof. As such, the appropriate scope of the invention is to be determined according to the claims.

Although various embodiments which incorporate the teachings of the present invention have been shown and described in detail herein, those skilled in the art can readily devise many other varied embodiments that still incorporate these teachings. Thus, while the foregoing is directed to various embodiments of the present invention, other and further embodiments of the invention may be devised without departing from the basic scope thereof. As such, the appropriate scope of the invention is to be determined according to the claims.

## Claims

1. A blood pump, comprising:
a cannula having a lumen therethrough;
an inflow cage coupled to a distal end of the cannula, the inflow cage comprising an inflow check valve that is configured to allow blood to flow into the lumen only when a pressure inside the lumen is less than a pressure of blood external to the inflow check valve;
an outflow cage coupled to a proximal end of the cannula, the outflow cage comprising an outflow check valve that is configured to allow blood to flow out of the lumen through at least one outlet only when a pressure in the lumen is greater than a pressure of blood external to the outflow check valve;
a catheter operably coupled to a proximal end of the outflow cage; and
a pump capable of generating positive and negative pressures operably coupled to the lumen.

2. The blood pump of claim 1, wherein the pump is coupled to a proximal end of the catheter.

3. The blood pump of claim 2, wherein the pump is configured to be positioned external to a patient during operation.

4. The blood pump of claim 3, wherein the pump is a piston pump.

5. The blood pump of any one of claims 1-4,
further comprising a purge pump configured to provide a purge fluid to the pump; and/or
further comprising a distal extension assembly operably connected to a distal end of the inflow cage.

6. The blood pump of claim 5, wherein the distal extension assembly comprises a check valve configure to allow blood to flow through a distal end of the inflow cage.

7. The blood pump of any one of claims 1-6, wherein the inflow check valve comprises a membrane operatively coupled to an internal surface of the inflow cage.

8. The blood pump of claim 7, wherein the membrane is integrally formed with the inflow check valve.

9. The blood pump of any one of claims 1-8, wherein the inflow check valve comprises an elastomer reinforced with nitinol.

10. The blood pump of any one of claims 1-9, wherein the outflow check valve comprises a membrane coupled to an external surface of the outflow cage.

11. The blood pump of claim 10,
wherein the membrane is integrally formed with the outflow check valve; and/o
wherein the membrane extends over the at least one outlet and at least a portion of the catheter.

12. The blood pump of any one of claims 1-11,
wherein the outflow check valve comprises an elastomer reinforced with nitinol;
wherein the blood pump further comprises a sleeve coupled to an external surface of the blood pump, the sleeve being configured to extend over the outflow check valve and at least a portion of the catheter, the sleeve having a proximal outlet configured to allow blood to exit the sleeve;
wherein the catheter comprises an inner surface having a low-friction coating; and/or
wherein the blood pump is configured to transfer blood through the cannula at a maximum rate of 1-5 L/min through the cannula.

13. A system, comprising:
a blood pump of any one of claims 1-12; and
a controller operably connected to the blood pump.

14. A method for using a blood pump, comprising:
providing a blood pump of any one of claims 1-12;
priming the blood pump; and
inserting the blood pump into a patient through at least one blood vessel.

15. The method of claim 14, wherein the blood pump comprises a piston pump including a piston and a cylinder, and wherein the method further comprises repeatedly:
moving the piston so as to generate a negative pressure, closing the outflow check valve and opening the inflow check valve, drawing blood through the inflow check valve and into the cylinder; and
moving the piston so as to generate a positive pressure, closing the inflow check valve and opening the outflow check valve, forcing blood out of the cylinder and through the outflow check valve.
